# EUROPÄISCHE PATENTANMELDUNG

(11) **EP 1 477 201 A1**
(43) Veröffentlichungstag der Anmeldung: **17.11.2004**
(21) Anmeldenummer: 04010598.3
(22) Anmeldetag: 04.05.2004
(51) Int. Cl.: A61M 16/12

(54) **Verfahren und Vorrichtung zur kontrollierten Zumischung eines Gases oder Gasgemisches zu einem Gas(gemisch)strom**

(30) Priorität: 13.05.2003 DE 10321448
(71) Anmelder: INO Therapeutics GmbH, 2345 Brunn/Gebirge (AT)
(72) Erfinder: Fritz, Markus, 2340 Mödling (AT); Krebs, Christian, 2331 Vösendorf (AT); Müllner, Rainer, 2351 Wiener Neudorf (AT); Romako, Christian, 3382 Loosdorf (AT)
(74) Vertreter: Kasseckert, Rainer

(57) **Zusammenfassung**

Es werden ein Verfahren sowie eine Vorrichtung zur kontrollierten Zumischung wenigstens eines Gases oder Gasgemisches zu einem Gas(gemisch)strom, so dass das Gas oder Gasgemisch nach der Zumischung zu dem Gas(gemisch)strom in diesem eine voreingestellte und/oder voreinstellbare Konzentration aufweist, beschrieben, wobei die Zumischung mit Hilfe von einer Druckregelvorrichtung (V) erfolgt.

## Beschreibung

Die Erfindung betrifft gemäß einer ersten Altemative ein Verfahren zur kontrollierten Zumischung wenigstens eines Gases oder Gasgemisches zu einem Gas(gemisch)strom, so dass das Gas oder Gasgemisch nach der Zumischung zu dem Gas(gemisch)strom in diesem eine voreingestellte und/oder voreinstellbare Konzentration aufweist, wobei
a) der Volumenstrom und/oder Druck des Gas(gemisch)stromes ermittelt wird,
b) der Ist-Wert des Volumenstromes und/oder Druckes des zuzumischenden Gases oder Gasgemisches ermittelt wird,
c) in Abhängigkeit von dem ermittelten Volumenstrom und/oder Druck des Gas(gemisch)stromes der Soll-Wert des Volumenstromes und/oder Druckes des zuzumischenden Gases oder Gasgemisches errechnet und mit dem aktuellen Ist-Wert verglichen wird, und
d) in Abhängigkeit von der Differenz zwischen dem Soll- und dem Ist-Wert eine Anpassung des Ist-Wertes an den Soll-Wert erfolgt.

Gemäß einer zweiten Alternative betrifft die Erfindung ein Verfahren zur kontrollierten Zumischung einer voreingestellten und/oder voreinstellbaren Menge (Soll-Wert) eines Gases oder Gasgemisches zu einem Gas(gemisch)strom, wobei
a') ggf. der Volumenstrom und/oder Druck des Gas(gemisch)stromes ermittelt wird,
b') der Ist-Wert des zuzumischenden Gases oder Gasgemisches mit dem Soll-Wert verglichen wird, und
c') in Abhängigkeit von der Differenz zwischen dem Soll- und dem Ist-Wert eine Anpassung des Ist-Wertes an den Soll-Wert erfolgt.

Ferner betrifft die Erfindung zu den beiden vorgenannten Verfahrensalternativen korrespondierende Vorrichtungen gemäß den Oberbegriffen der unabhängigen Patentansprüchen 11 und 12.

Gattungsgemäße Verfahren sowie Vorrichtungen finden bspw. bei der maschinellen Beatmung von Personen bzw. Patienten, bei spontanatmenden Patienten, bei der Anästhesie sowie bei unterschiedlichsten Atemtherapien, bei denen dem Patienten zu Therapiezwecken ein Gas oder Gasgemisch zugeführt wird, Anwendung.

Beispielhaft erwähnt sei die kontrollierte Zudosierung von NO zu einem Atemgas. Stickstoffmonoxid bzw. NO ist mittlerweile als Medikament gegen pulmonale Hypertension bekannt, also als Vasodilator. Der Vorteil der Behandlung mit den bereits vielfach beschriebenen NO-Gemischen ist, dass NO nur lokal, also im pulmonalen Kreislauf, und nicht systemisch wirksam ist. Das NO bzw. NO-Gasgemisch wird dem Patienten per Inhalationen appliziert. Dies bedeutet, dass das therapeutisch wirksame Gas dem Atemgas, das dem Patienten zugeführt wird, zugemischt wird.

Bei der Zumischung von Gasen oder Gasgemischen - nachfolgend nunmehr als "Gas" bezeichnet - zu einem Gas(gemisch)strom - nachfolgend als "Atemgasstrom" bezeichnet- stellt im Regelfall die sich nach der Zumischung des Gases ergebende Konzentration des zuzumischenden Gases in dem Atemgasstrom denjenigen Parameter dar, der vom Arzt eingestellt wird. Der Parameter Konzentration soll bzw. muss im Regelfall während der Behandlung bzw. Therapie konstant gehalten werden.

Kommt es nunmehr zu einer Veränderung des Atemgasvolumenstromes, so ist auch eine Veränderung des zuzumischenden Gasstromes vorzunehmen, da ansonsten die gewünschte und eingestellte Konzentration in dem Atemgasstrom nicht konstant bliebe. Die Menge bzw. das Volumen des zuzumischenden Gases kann nunmehr entweder durch eine Variation des Durchflusses bzw. der Dauer der Zumischung bzw. Dosierung verändert werden.

Alternativ zu der vorbeschriebenen Verfahrensweise gibt es eine Vielzahl von Anwendungsfällen, bei denen den Patienten eine bestimmte Dosis eines Gases dem Atemgasstrom zugemischt werden soll. In diesen Fällen wird gemäß der zweiten Alternative des gattungsgemäßen Verfahrens verfahren.

Bei der Behandlung von Patienten gilt es ferner zu berücksichtigen, dass im Regelfall möglichst ausschließlich während der Inspirationsphase dem Atemgasstrom Gas zugemischt wird. Im Falle eines konstanten und kontinuierlichen Atemgasstromes kann - in Abhängigkeit von der gewünschten bzw. eingestellten Konzentration oder Dosis - lediglich der Durchfluss des zuzumischenden Gases variiert werden, da die Zumischung ebenfalls kontinuierlich durchgeführt werden muss. Kommt es zu einer Veränderung des kontinuierlichen Atemgasstromes, so muss - um weiterhin die eingestellte Konzentration beibehalten zu können - der Durchfluss des zuzumischenden Gases entsprechend erhöht oder erniedrigt werden.

Bisher erfolgt die Anpassung des Ist-Wertes an den Soll-Wert dadurch, dass der Durchfluss des zuzumischenden Gases oder Gasgemisches mittels eines Ventiles variiert wird.

Hierbei ist von Nachteil, dass Vordruckschwankungen den Ist-Durchfluss beeinflussen. Dies bedeutet, dass sich bei jeder Vordruckschwankung auch der Ist-Durchfluss verändert und folglich durch eine Verstellung des Ventiles nachgeregelt werden muss.

Aufgabe der vorliegenden Erfindung ist es, gattungsgemäße Verfahren sowie gattungsgemäße Vorrichtungen zur kontrollierten Zumischung wenigstens eines Gases oder Gasgemisches zu einem Gas(gemisch)strom bzw. Atemgasstrom anzugeben, das bzw. die es ermöglicht- unabhängig von den vorgenannten, gewünschten Anwendungszwecken - während einer Behandlung bzw. Therapie zu jedem beliebigen Zeitpunkt die notwendige, zuzumischende Gasmenge bereitzustellen bzw. an die jeweils herrschenden Gegebenheiten, insbesondere an den Volumenstrom und/oder Druck des dem Patienten zuzuführenden Gas(gemisch)stromes bzw. Atemgasstromes, anzupassen.

Verfahrensseitig wird diese Aufgabe dadurch gelöst, dass die Anpassung des Ist-Wertes an den Soll-Wert mittels einer den Druck des zuzumischenden Gases oder Gasgemisches beeinflussenden Druckregelvorrichtung erfolgt.

Die erfindungsgemäßen Vorrichtungsalternativen zur kontrollierten Zumischen wenigstens eines Gases oder eines Gasgemisches zu einem Gas(gemisch)strom bzw. Atemgasstrom sind dadurch gekennzeichnet, dass die Regelvorrichtung eine Druckregelvorrichtung ist.

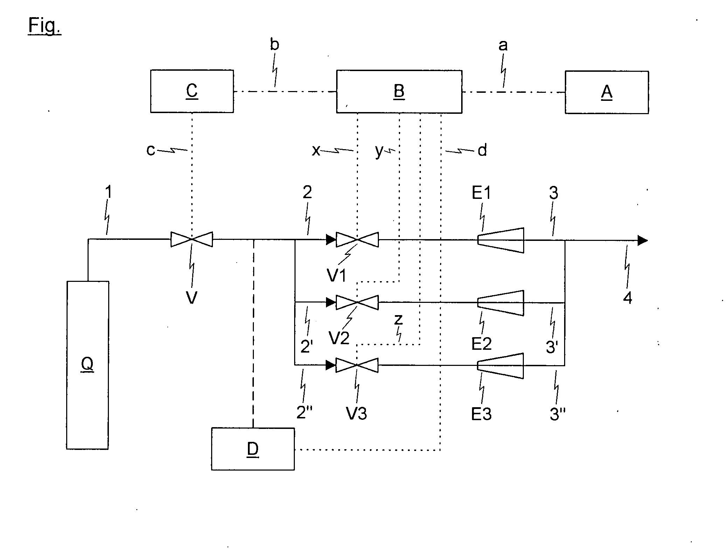

Die Erfindung sowie weitere Ausgestaltungen derselben sei nachfolgend anhand des in der Figur dargestellten Ausführungsbeispieles näher erläutert.

Die Figur zeigt lediglich schematisiert dargestellt ein mögliches Ausführungsbeispiel der erfindungsgemäßen Vorrichtung, mittels derer die erfindungsgemäßen Verfahrensalternativen realisierbar sind.

In der Figur dargestellt ist eine erste Messeinheit A, die der Erfassung des Volumenstromes und/oder des Druckes des Gas(gemisch)stromes bzw. Atemgasstromes, der beispielsweise einem Patienten zu Therapiezwecken zugeführt wird, dient. In der Figur nicht dargestellt ist diejenige Leitung, über die dem Patienten der Atemgasstrom zugeführt wird.

Die Messeinheit A steht über eine Datenleitung a mit einer Auswerteeinheit B in Verbindung. Die Messeinheit A kann als Durchfluss- bzw. Volumenstrommesseinheit ausgebildet sein. Sie kann alternativ oder ergänzend auch als Drucksensor ausgebildet sein, der beispielsweise den Unterdruck, der während der Inspirationsphase entsteht, erfasst.

Des Weiteren ist eine Messeinheit D vorgesehen, die der Erfassung des Volumenstromes und/oder Druckes des zuzumischenden Gases bzw. Gasgemisches in der Leitung 1 dient. Auch die Messeinheit D steht über eine Datenleitung d mit der bereits erwähnten Auswerteeinheit B in Verbindung.

Das dem Atemgasstrom zuzumischende Gas bzw. Gasgemisch stammt aus einer entsprechend geeigneten "Gasquelle" Q - hierbei handelt es sich beispielsweise um eine Gasflasche oder -patrone - und wird über die Leitungen 1, 2, 3 und 4, dem dem Patienten zuzuführenden Atemgasstrom beigemischt.

Erfindungsgemäß ist nunmehr in der Leitung 1 eine Druckregelvorrichtung V vorgesehen; diese wird beispielsweise über einen elektrischen Stell- oder Schrittmotor C angesteuert (c). Auch der Elektromotor C steht über eine Datenleitung b mit der Auswerteeinheit B in Verbindung.

In der Auswerteeinheit B wird in Abhängigkeit von dem mittels der Messeinheit A ermittelten Volumenstrom und/oder Druck des Gas(gemisch)stromes der Soll-Wert des Volumenstromes und/oder Druckes des zuzumischenden Gases oder Gasgemisches errechnet und mit dem aktuellen, mittels der Messeinheit D ermittelten Ist-Wert des Volumenstromes und/oder Druckes des zuzumischenden Gases oder Gasgemisches verglichen. In Abhängigkeit von der Differenz zwischen dem Soll- und dem Ist-Wert erfolgt sodann eine Anpassung des Ist-Wertes an den Soll-Wert, indem mittels der dem zuzumischenden Gas- oder Gasgemischstrom zugeordneten, von der Auswerteeinheit B ansteuerbaren Druckregelvorrichtung V der Volumenstrom und/oder Druck des zuzumischenden Gases oder Gasgemisches entsprechend verändert wird.

Sofern - entsprechend einer weiteren Ausgestaltung des erfindungsgemäßen Verfahrens - die Druckregelvorrichtung V von einem Stell- oder Schrittmotor C angetrieben wird und anhand der jeweiligen Position des Stell- oder Schrittmotors C der voreingestellte Druck der Druckregelvorrichtung V bekannt ist, erübrigt sich eine zusätzliche Erfassung des Durchfluss- und/oder Druck-Ist-Wertes des zuzumischenden Gases oder Gasgemisches, da aus dem voreingestellten Druck und der bekannten Geometrie der Druckregelvorrichtung V auf den Durchfluss des zuzumischenden Gases oder Gasgemisches geschlossen werden kann.

Des Weiteren ist in der Leitung 2 ein Ventil V1 vorgesehen, das ebenfalls über eine Datenleitung x mit der Auswerteeinheit B in Wirkverbindung steht bzw. von dieser angesteuert wird. Das Ventil V1 dient dazu, den zuzumischenden Gasstrom jederzeit zu unterbrechen bzw. (wieder) freizugeben.

Im Falle eines nicht kontinuierlichen Atemgasstromes werden mittels der Messeinheit A die Inspirations- und Expirationsphasen des Patienten erkannt. Entsprechend dieser Phasen wird dann der über die Leitungen 1, 2, 3 und 4 dem Atemgasstrom zuzumischende Gasstrom mittels des Ventils V1 unterbrochen bzw. freigegeben. In Abhängigkeit von der jeweils gewünschten Dosierform kann das Ventil V1 ausschließlich während der Inspirationsphase, ausschließlich während der Expirationsphase oder in beliebigen Kombinationen der vorgenannten Verfahrensweisen freigegeben werden. So ist mit dem erfindungsgemäßen Verfahren beispielsweise eine inspirationssynchronisierte Beatmung eines Patienten realisierbar.

Ferner kann - entsprechend einer weiteren vorteilhaften Ausgestaltung der Erfindung - eine dem Ventil V1 nachgeschaltete Düse E1 in der Leitung 3 vorgesehen werden. Aufgrund der bekannten Geometrie der Düse E1 kann bei einem bekannten Vordruck der Durchfluss exakt berechnet werden. Über die Leitung 4 wird das zuzumischende Gas der in der Figur nicht dargestelltes Atemgasstromleitung und anschließend unmittelbar dem Patienten zugeführt.

Wie in der Figur dargestellt, können - der Messeinheit D nachgeschaltet - auch mehrere, zu den Leitungen 2 und 3 parallel angeordnete Leitungen 2' und 3' bzw. 2" und 3" angeordnet werden, wobei diese jeweils ebenfalls Ventile V2 bzw. V3 sowie Düsen E2 bzw. E3 aufweisen. Die Ansteuerung der Ventile V2 und V3 durch die Auswerteeinheit B erfolgt über die Datenleitungen y bzw. z.

Diese Ausführungsform der Erfindung ermöglicht es, insbesondere bei der Verwendung von Düsen mit unterschiedlichen Düsengeometrien größere Dosierbereiche zu realisieren. Auch können zeitgleich mehrere der Ventile V1, V2 bzw. V3 geöffnet werden, wodurch sich die Volumenströme durch die einzelnen Ventile V1, V2 bzw. V3 bzw. Leitungen 2' und 3' bzw. 2" und 3" addieren.

Auch kann mittels dieser Verfahrensweise der Druckabfall, der bei dem Öffnen eines Ventils V1, V2 bzw. V3 zwangsläufig entsteht und damit zu einer Reduzierung des Durchflusses führt, kompensiert werden, indem der Druck vor dem Öffnen des entsprechenden Ventils V1, V2 bzw. V3 höher eingestellt wird, um auf diese Weise nach dem Öffnen des Ventils V1, V2 bzw. V3 den gewünschten Durchfluss zu erhalten. Um dies zu erreichen, kann beispielsweise auch die Öffnungszeit des Ventils V1, V2 bzw. V3 entsprechend verlängert werden.

Wie eingangs erwähnt eignen sich die erfindungsgemäßen Verfahrens- sowie Vorrichtungsalternativen für die maschinelle Beatmung von Patienten, für spontanatmende Patienten, für die Anästhesie sowie für unterschiedlichste Atemtherapien, bei denen dem Patienten zu Therapiezwecken ein Gas oder Gasgemisch zugeführt wird.

Neben der ebenfalls bereits erwähnten Zudosierung von NO zu einem Atemgas, wobei NO als Medikament gegen pulmonale Hypertension wirkt, kann das zuzumischende Gas oder Gasgemisch Luft, Perflurcarbon, O₂, Xe, ein Xe-Gemisch, ein NO-Gemisch, CO, ein CO-Gemisch, ein CO₂-Gemisch zur Atemstimulierung, ein H₂-Gemisch, N₂O, ein N₂O-Gemisch, ein SF₆-Gemisch, Nitrosoethanol oder ein Anästhesiegas sein. Der dem Patienten zuzuführende Gas(gemisch)- bzw. Atemgasstrom ist vorzugsweise Luft, O₂, N₂, Ar, Xe, He, SF₆ und/oder ein Anästhesiegas, ein Gemisch aus diesen Gasen oder ein anderes therapeutisch oder therapeutisch nicht wirksames Gas oder Gasgemisch.

## Patentansprüche

1. Verfahren zur kontrollierten Zumischung wenigstens eines Gases oder Gasgemisches zu einem Gas(gemisch)strom, so dass das Gas oder Gasgemisch nach der Zumischung zu dem Gas(gemisch)strom in diesem eine voreingestellte und/oder voreinstellbare Konzentration aufweist, wobei
a) der Volumenstrom und/oder Druck des Gas(gemisch)stromes ermittelt wird (A),
b) der Ist-Wert des Volumenstromes und/oder Druckes des zuzumischenden Gases oder Gasgemisches ermittelt wird (D),
c) in Abhängigkeit von dem ermittelten Volumenstrom und/oder Druck des Gas(gemisch)stromes der Soll-Wert des Volumenstromes und/oder Druckes des zuzumischenden Gases oder Gasgemisches errechnet und mit dem aktuellen Ist-Wert verglichen wird (B), und
d) in Abhängigkeit von der Differenz zwischen dem Soll- und dem Ist-Wert eine Anpassung des Ist-Wertes an den Soll-Wert erfolgt,
**dadurch gekennzeichnet, dass** die Anpassung des Ist-Wertes an den Soll-Wert mittels einer den Druck des zuzumischenden Gases oder Gasgemisches beeinflussenden Druckregelvorrichtung (V) erfolgt.

2. Verfahren zur kontrollierten Zumischung einer voreingestellten und/oder voreinstellbaren Menge (Soll-Wert) eines Gases oder Gasgemisches zu einem Gas(gemisch)strom, wobei
a') der Ist-Wert des zuzumischenden Gases oder Gasgemisches mit dem Soll-Wert verglichen wird, und
b') in Abhängigkeit von der Differenz zwischen dem Soll- und dem Ist-Wert eine Anpassung des Ist-Wertes an den Soll-Wert erfolgt,
**dadurch gekennzeichnet, dass** die Anpassung des Ist-Wertes an den Soll-Wert mittels einer den Druck des zuzumischenden Gases oder Gasgemisches beeinflussenden Druckregelvorrichtung erfolgt.

3. Verfahren nach Anspruch 2, **dadurch gekennzeichnet, dass** der Volumenstrom und/oder Druck des Gas(gemisch)stromes ermittelt wird.

4. Verfahren nach einem der vorhergehenden Ansprüche 1 bis 3, **dadurch gekennzeichnet, dass** die Druckregelvorrichtung (V) von einem Stell- oder Schrittmotor (C) angetrieben wird.

5. Verfahren nach einem der vorhergehenden Ansprüche 1 bis 4, wobei die Druckregelvorrichtung von einem Stell- oder Schrittmotor angetrieben wird und anhand der jeweiligen Position des Stell- oder Schrittmotors der voreingestellte Druck der Druckregelvorrichtung bekannt ist, **dadurch gekennzeichnet, dass** eine zusätzliche Erfassung des Durchfluss- und/oder Druck-Ist-Wertes des zuzumischenden Gases oder Gasgemisches unterbleibt.

6. Verfahren nach einem der vorhergehenden Ansprüche 1 bis 5, **dadurch gekennzeichnet, dass** die Zumischung des Gases oder Gasgemisches (1) zu dem Gas(gemisch)strom zumindest zeitweilig unterbrochen wird.

7. Verfahren nach einem der vorhergehenden Ansprüche 1 bis 6, wobei das Gas oder Gasgemisch einem einer Person zuzuführenden Gas(gemisch)strom zugemischt wird, **dadurch gekennzeichnet, dass** zumindest während der Expirationsphase der Person die Zumischung des Gases oder Gasgemisches unterbrochen wird.

8. Verfahren nach einem der vorhergehenden Ansprüche 1 bis 7, **dadurch gekennzeichnet, dass** der zuzumischende Gas- oder Gasgemischstrom (1) auf mehrere Teilströme (2, 2', 2"), deren Durchflüsse regelbar sind, aufgeteilt wird.

9. Verfahren nach einem der vorhergehenden Ansprüche 1 bis 8, **dadurch gekennzeichnet, dass** das zuzumischende Gas oder Gasgemisch Luft, Perflurcarbon, O₂, Xe, ein Xe-Gemisch, NO, ein NO-Gemisch, CO, ein CO-Gemisch, ein CO₂-Gemisch zur Atemstimulierung, ein H₂-Gemisch, N₂O, ein N₂O-Gemisch, ein SF₆-Gemisch, Nitrosoethanol oder ein Anästhesiegas ist.

10. Verfahren nach einem der vorhergehenden Ansprüche 1 bis 9, **dadurch gekennzeichnet, dass** der Gas(gemisch)strom Luft, O₂, N₂, Ar, Xe, He, SF₆ und/oder ein Anästhesiegas, ein Gemisch aus diesen Gasen oder ein anderes therapeutisch oder therapeutisch nicht wirksames Gas oder Gasgemisch ist.

11. Vorrichtung zur kontrollierten Zumischung wenigstens eines Gases oder Gasgemisches zu einem Gas(gemisch)strom, so dass das Gas oder Gasgemisch nach der Zumischung zu dem Gas(gemisch)strom in diesem eine voreingestellte und/oder voreinstellbare Konzentration aufweist, aufweisend
a) eine Messeinheit (A), die der Erfassung des Volumenstromes und/oder Druckes des Gas(gemisch)stromes dient,
b) eine Messeinheit (D), die der Erfassung des Ist-Wertes des Volumenstromes und/oder Druckes des zuzumischenden Gases oder Gasgemisches (1, 2, 2', 2") dient,
c) eine Auswerteeinheit (B), in der in Abhängigkeit von dem ermittelten Volumenstrom und/oder Druck des Gas(gemisch)stromes der Soll-Wert des Volumenstromes und/oder Druckes des zuzumischenden Gases oder Gasgemisches errechnet und mit dem aktuellen Ist-Wert verglichen wird, und
d) eine dem zuzumischenden Gas- oder Gasgemischstrom (1) zugeordnete Regelvorrichtung, die von der Auswerteeinheit (B) derart ansteuerbar ist bzw. angesteuert wird, dass in Abhängigkeit von der Differenz zwischen dem Soll- und dem Ist-Wert eine Anpassung des Ist-Wertes an den Soll-Wert erfolgt,
**dadurch gekennzeichnet, dass** die Regelvorrichtung eine Druckregelvorrichtung (V) ist.

12. Vorrichtung zur kontrollierten Zumischung einer voreingestellten und/oder voreinstellbaren Menge (Soll-Wert) eines Gases oder Gasgemisches zu einem Gas(gemisch)strom, aufweisend
a') eine Messeinheit (D), die der Erfassung des Ist-Wertes des Volumenstromes des zuzumischenden Gases oder Gasgemisches (1, 2, 2', 2") dient,
b') eine Auswerteeinheit (B), in der der ermittelte Ist-Wertes des Volumenstromes mit dem Soll-Wert verglichen wird, und
c') eine dem zuzumischenden Gas- oder Gasgemischstrom (1) zugeordnete Regelvorrichtung, die von der Auswerteeinheit (B) derart ansteuerbar ist bzw. angesteuert wird, dass in Abhängigkeit von der Differenz zwischen dem Soll- und dem Ist-Wert eine Anpassung des Ist-Wertes an den Soll-Wert erfolgt,
**dadurch gekennzeichnet, dass** die Regelvorrichtung eine Druckregelvorrichtung (V) ist.

13. Vorrichtung nach Anspruch 12, **dadurch gekennzeichnet, dass** sie eine Messeinheit (A), die der Erfassung des Volumenstromes und/oder Druckes des Gas(gemisch)stromes dient, aufweist.

14. Vorrichtung nach einem der vorhergehenden Ansprüche 11 bis 13, **dadurch gekennzeichnet, dass** der Druckregelvorrichtung (V) ein oder mehrere, vorzugsweise parallel zueinander angeordnete Ventile (V1, V2, V3) nachgeschaltet sind.

15. Vorrichtung nach einem der vorhergehenden Ansprüche 11 bis 14, **dadurch gekennzeichnet, dass** der Druckregelvorrichtung (V) eine oder mehrere, vorzugsweise parallel zueinander angeordnete Düsen (E1, E2, E3) nachgeschaltet sind.

16. Vorrichtung nach einem der vorhergehenden Ansprüche 11 bis 15, **dadurch gekennzeichnet, dass** die Druckregelvorrichtung (V) mittels eines Stell- oder Schrittmotors (C) angetrieben wird.
